**Europäisches Patentamt**

**European Patent Office**

**Office européen des brevets**

(11) Publication number: **0 110 502**
**B1**

(12) **EUROPEAN PATENT SPECIFICATION**

(45) Date of publication of patent specification: **07.02.90**

(21) Application number: **83304741.8**

(22) Date of filing: **16.08.83**

(51) Int. Cl.⁵: **A 61 K 9/48, A 61 J 3/07**

(54) Foam capsules and their production.

(30) Priority: **29.10.82 US 438147**

(43) Date of publication of application:
**13.06.84 Bulletin 84/24**

(45) Publication of the grant of the patent:
**07.02.90 Bulletin 90/06**

(84) Designated Contracting States:
**BE DE FR GB IT**

(56) References cited:
**FR-A-2 135 022**
**US-A-4 331 547**

(73) Proprietor: **WARNER-LAMBERT COMPANY**
**201 Tabor Road**
**Morris Plains New Jersey 07950 (US)**

(72) Inventor: **Mayer, Jean Philippe**
**59, rue des Trois-Chateaux**
**F-68000 Colmar (FR)**
Inventor: **Wittwer, Fritz**
**Bündtenstrasse 11**
**CH-4411 Lupsingen (CH)**

(74) Representative: **Jones, Michael Raymond et al**
**HASELTINE LAKE & CO. Hazlitt House 28**
**Southampton Buildings Chancery Lane**
**London WC2A 1AT (GB)**

Courier Press, Leamington Spa, England.

**Description**

This invention relates to the production of foam capsules, and the capsules produced thereby.

According to the present invention, there is provided a process for the production by the dip moulding technique of a cap portion and/or body portion suitable for forming a foam capsule with telescopically engageable body and cap portions, which process comprises the following steps:—

(A) dissolving gelatin in water to make an aqueous gelatin solution, such that the weight of gelatin in the aqueous gelatin solution is in the range from 10 to 50% by weight of the solution, and the pH of the gelatin solution is in the range from 3.0 to 10.0;

(B) introducing a gas into the aqueous gelatin solution to make a gas-containing aqueous gelatin solution;

(C) micronizing the gas in the gas-containing gelatin solution to make a micronized gas-containing aqueous gelatin solution; and

(D) forming by the dip moulding technique foam capsule body and/or cap portion(s) from the micronized gas-containing aqueous gelatin solution.

References are made herein to "micronizing"; a micron is one millionth of a metre, i.e. a micrometre, but this strict dimensional definition is not necessarily to apply to the act of micronizing or to equipment such as a micropipe, even though certain bubbles produced in the process of the present invention may have, but need not have, a diameter as small as one micrometre. Similar remarks apply to terms such as "microdispersion" which appear herein. More particularly, the invention relates to pharmaceutically acceptable capsules having capsule body and cap portions, formed by dipmolding, using a film-forming mixture obtained by a microdispersion of a suitable gas in a gelatin solution.

As used herein, the term "gelatin" means gelatin and derivatives thereof.

As used herein, the term "gelatin foam" means a homogeneous mixture obtained by microdispersion of a gas in a gelatin solution.

The term "capsules" means hard shell capsules having telescopically engaged body and cap portions obtained by a dipmolding technique (see U.S. Patent No. 3,173,840.)

The term "foam capsules" means such capsules, obtained by dipmolding into gelatin foams, the wall of which being formed by a homogeneous microdispersion of gas in dry gelatin.

As is known, capsules are a preferred form of administration for medicaments and similar products. However, in many cases, the disintegration speed of the gelatin capsules can vary considerably depending on the composition of the contents. For example, the disintegration of capsules containing a lipophilic medicament is delayed because of the lipophilic properties of the content. The rapid release of the medicament is thereby impaired which can have a detrimental effect on its bioavailability. In order to avoid these disadvantages, attempts have been made to ensure a rapid release of the capsule contents by means of a suitable form of the capsule walls, for example by providing them with holes or other apertures, as described in U.S. Patent No. 3,620,759. Such capsules have, however, the disadvantage that, in the case of pulverulent contents, premature release of the contents takes place, during storage or transport. In order to avoid this undesirable, premature release of the capsules' contents, capsules of this type, having holes or other apertures, have been provided completely or partially with a coating of a water-soluble material, as described in U.S. Patent No. 3,823,816. Although the undesirable, premature release of the capsules' contents can be largely avoided in this manner, the production of such capsule is difficult and expensive.

The objective of this invention was therefore to modify the capsule wall of gelatin capsules provided for medicinal and other purposes in such a manner that a control, particularly an acceleration, of the capsule disintegration can be achieved in a simple manner.

On the other hand, many additives used in pharmaceutical materials are now being critically examined. For example, titanium dioxide, commonly used as an opacifier in hard shell gelatin capsules, is under examination as to whether or not it is pharmaceutically acceptable.

A new capsule type, the wall of which would be opaque without addition of titanium dioxide or other similar chemicals and which could therefore contain only natural and biodegradable products, would have important advantages over conventional hard shell gelatin capsules.

It has been found in the present invention that it is possible to obtain white opaque film-forming mixtures by simple inclusion, followed by micronization, of suitable gases such as air, oxygen, nitrogen, carbon dioxide and argon into natural transparent gelatin solutions. The opacity and white color shade of the foam capsules obtained by dipmolding into such film-forming mixtures, is a function of the included gas quantity and of its micronization level.

It has also been found in the present invention that the stability of the foam increased in the following order or microdispersion of gases: carbon dioxide, oxygen, air, nitrogen and argon. However, economic considerations favor the use of air and nitrogen.

It has also been found that the particular structure of the wall of such foam capsules, assures, with regard to conventional hard shell gelatin capsules with a same wall thickness, a notable increase of the disintegration speed. This disintegration time can be varied by modifying both gas content and thickness of the wall. Increasing the gas content and/or decreasing the wall thickness, the disintegration time is reduced. Decreasing the gas content and/or increasing the wall thickness, the disintegration time is increased.

EP 0 110 502 B1

As a result of these discoveries, it was possible to achieve the objectives of the present invention and to provide a hard shell gelatin capsule, the wall of which is opaque without additives such as titanium dioxide, that decomposes at a particularly higher speed which can be controlled and which is suitable for medicinal purposes. This foam capsule is characterized in that the material forming the capsule wall is a foam obtained by microdispersion of a suitable gas in gelatin, optionally with the inclusion of a plasticizer and/or a coloring agent, and/or a flavoring agent, and/or a foam stabilizer, and/or a gelatin extender.

The gelatin foams, suitable to obtain foam capsules according to the present invention, are made from aqueous solutions of gelatin comprising between 10 to 50% by weight of gelatin, better in the range of 15 to 35% and best in the range of 21 to 28%. Different grades of pure gelatins or mixtures thereof can be used at pH values betwen 3.0 to 10.0, with better results in the range of pH 5.0 to 9.0.

The foam may be produced according to any of a number of common methods, wherein air, or a gas such as nitrogen, oxygen, argon, carbon dioxide or another suitable gas, or a mixture thereof, is mixed with the gelatin solution and micronized. The most suitable foaming devices are based on a mechanical action, such as obtained for example with a high-speed blender, or a baker's "whip" in which a wire whisk is both rotated and travels in a circular path, or with a centrifugal emusifying device in which a rotor, rotating at high speed, forces the gas/gelatin mixture through a performated stator where the bubbles are micronized. These devices can be used for batch foam production or most preferably in line, in systems where the foam flows continuously through a dish, where dipmolding is performed and is recirculated through a foaming reservoir wherein the gas/gelatin mixture is continuously micronized and where regulation of the optimal gas/water/gelatin content takes place. In-line foaming can be done in an open reservoir fitted with a gas supply and, for example, with a centrifugal emulsifier, in which case the diameter of the micronized gas bubbles is in a considerable measure controlled by the rotation speed of the rotor and the dimensions of the perforations of the stator and the gap between rotor and stator. Foaming can also be done in a closed chamber such as in a continuous pressure beater, commonly used in the food industry for aerated desserts or marshmallow confection. In the latter case, the foam is obtained by simultaneous injection of a controlled quantity of gas and gelatin solution into a mixing head formed by a rotor and a stator, both being fitted with a high number of teeth to accomplish micronization. The gas/gelatin ratio in the foam is controlled by the corresponding injection rates. The micronization level of the gas bubbles is, in a considerable measure, controlled by the number of teeth, the chamber configuration and the contact time of the foam in the mixing head.

Other suitable foam generation systems may be used such as aerosol generation, ultrasonic emulsifying, injecting gas into gelatin solution through micropipes, sintered glass or sintered metal, or passing of the gas/gelatin solution mixture through tubes packed with polyamide fibers or steel wool (as described in Netherlands Patent Application No. 7,609,307).

It is recommended to equip all the above devices with a heating system in order to avoid premature gelling of the foam.

Suitable foams for obtaining foam capsules by dipmolding according to the present invention are characterized by a gas content between 8 to 70% vol/vol of the gelatin solution better between 13 to 31%; a density between 0.3 to 1.1 g/cm3, better between 0.7 to 0.95; a viscosity between 200 and 2,000 centipoises (0.2 and 2 Pa·s), better between 400 to 900 centipoises (0.4 to 0.9 Pa·s); and, for obtaining a desired opacity, by a diameter of the dispersed gas bubbles between 0.001 µm and 150 µm, better between 0.01 µm and 70 µm and best between 0.001 to 50 µm.

For capsule production, suitable metal mold pins are dipped in the conventional way into the gelatin foam and the wet film thus formed on the pins upon lifting from the foam is dried gradually to obtain the desired foam capsule parts. The wall thickness of capsules produced by dip-molding depends on the viscosity of the dipping solution. If a thin-walled capsule is desired, more water and/or less air are used in order to lower the viscosity, whereas if a thick-walled capsule is desired, less water and/or more air are added in order to increase the viscosity.

The wall thickness of the capsule is also dependent on the temperature of the dipping mixture. Depending upon the desired wall thickness and on the foam composition, the foam in the dipping dish is kept at a temperature between 30 to 65°C, better between 40 and 50°C. A condition for obtaining the foam capsule according to the present invention, is that the foam is sufficiently stable and durable, so that the uniformly dispersed gas bubbles are maintained while the foam is transferred from the foaming reservoir to the dipping dish.

A constant foam quality regulation is assured by simultaneous in-line viscosity and density measurements and subsequent addition of water and/or air and/or fresh gelatin solution.

In some cases, particularly when more diluted gelatin solutions are used, foam stabilizing agents may be added at varying concentrations. In general amounts of about 0.01 to 5% and preferably at 0.05 to 0.5% based upon the weight of gelatin have been found effective. Suitable illustrative foam stabilizing agents include, for example:

— viscosity increasing agents such as alginic acid and salts thereof, xanthan, cellulose derivatives such as carboxymethylcellulose, hydroxypropycellulose, hydroxypropylmethylcellulose, and microcrystalline cellulose;
— vegetable gums such as carrageenates, pectin, and agar;
— proteins such as albumin, and hydrolyzed animal proteins;

3

— esters such as sucrose esters, fatty acid esters and ethoxylates thereof, particularly polyglycerol and sorbitol derivatives thereof; glycerides and derivatives thereof such as succinyl monoglycerides; acetic-, lactic-, citric-, or acetylated tartaric acid esters of monoglycerides; glycosides, and derivatives thereof, such as glycosides; and lanolin and its derivatives;

— non-ionic surfactants such as fatty acid alkyolamides or ethoxylates thereof; amine oxides and particularly those containing long chain substituents such as N,N-dimethyldodecylamine, and substituted and unsubstituted long chain alcohols, such as lauryl alcohol, and sorbitol monooleate;

— anionic surfactants such as alkylarylsulphonates, particularly sodium dodecylbenzenesulfonic acid, and alcoholsulphates, particularly sodium lauryl sulphate, and ethersulphates;

— metal salts such as aluminum, calcium, potassium, and iron salts and mixtures thereof; and

— combinations of the above, particularly mixtures of egg albumin and microcrystalline cellulose or mixtures of non-ionic and anionic surfactants such as, for example, sodium lauryl sulphate and sorbitol monooleate or sodium lauryl sulphate and coconut fatty acid diethanolamide, and pharmaceutically acceptable.combinations of the foregoing compounds.

It must be noted, of course, that among the abovementioned stabilizers, only approved foam stabilizers may be used for the production of foam capsules for food or pharmaceutical uses.

For manufacturing hard shell foam capsules according to the present invention, the utilization of pharmaceutical goods coloring agents, flavoring agents and plasticizers leads to optimal product qualities without destroying or substantially altering their valuable physical properties.

Pharmaceutically acceptable coloring agents such as azo-dyes, as iron oxides and hydroxides, natural dyes etc. are optionally used. In general acceptable concentrations of between about 0.001 to 10%, better between 0.001 to 5% based upon weight of gelatin have been found effective.

Flavoring agents accepted for pharmaceutical and food use are usually prepared by or derived from synthetic flavor oils and/or oils derived from plants, leaves, flowers and fruits as well as combinations thereof. Representative flavor oils include peppermint oil, cinnamon oil, spearmint oil. Furthermore, natural or synthetic fruit flavors such as oils including lime, grape, orange, lemon, grapefruit and fruit essence including apple, pineapple, cherry and strawberry can be used.

The above flavoring agents are generally used at a concentration of about 0.01% to about 3% by weight on dry gelatin or derivatives thereof, better at about 0.2 to about 1.5%, and best at about 1.1%.

Plasticizers, particularly those of pharmaceutical grade, such as polyethylene glycol or preferably low molecular weight organic plasticizers, like glycerol, sorbitol, dioctyl-sodium sulfosuccinate, triethyl citrate, tributyl citrate, 1,2-propyleneglycol, mono-, di- and triacetates of glycerol may be utilized at various concentrations of between about 0.2 to 15% better between 0.2 to 5% based upon the weight of the gelatin.

In addition it has been found that the foam capsules of the present invention can be produced with various grades of gelatin combined with extenders of between about 2 to 40% content, by weight, better between about 5 to 20%, such as sunflower proteins, soybean proteins, cotton seed proteins, peanut proteins and rape seed proteins and better defatted qualities thereof. For manufacturing capsules with such gelatin extenders, and combinations thereof, the same kind of coloring agents, plasticizers, foam stabilizers and flavoring agents as described above, are suitable.

The foam capsules according to the present invention can be made, if desired with one or more locking features.

In the same manner as conventional hard shell gelatin capsules, foam capsules show optimal properties when the wall moisture content is between about 12 to 16%. In spite of the gas inclusion, the wall elasticity of foam capsules is similar to that of conventional hard shell gelatin capsules. An important advantage of the foam capsule is that it is considerably cheaper in manufacture than conventional hard shell gelatin capsules since a smaller amount of gelatin is required. For example, gelatin savings of between 40 to 50% can be reached without substantially altering the mechanical properties of foam capsules. In addition, since the particular structure of the wall of foam capsules provides a considerably increased exchange surface, an appreciable energy saving can be obtained during the manufacturing process because the wet half shells dry more easily and more rapidly on the mold bars than conventional hard gelatin capsule shells.

As mentioned above, the foam capsules have opaque walls, which effect is exclusively due to the homogeneous gas dispersion in the wall material and thus avoids the use of opacifying agents such as titanium dioxide or the like.

As described above, the possibility to obtain with foam capsules, within certain limits, controlled disintegration times and particularly shorter disintegration times than with conventional hard shell capsules, makes them suitable for a wide range of uses.

For example, capsules with moderate gas ratios in the wall, as obtained by dipping into gelatin foams having gas contents of between approximately 8 to 28% vol/vol, are useful for classical oral administration of medicaments. This means that they can be ingested without particular risk of premature opening in the mouth or in the esophagus. Their advantages over conventional hard shell gelatin capsules are a faster disintegration in the stomach, thereby maintaining an improved bioavailability of their contents, and less local irritations of the gastric mucous membrane may be obtained.

On the other hand, foam capsules with higher gas ratios in the wall are not adapted for classical oral administrations since the disintegration is so fast that a premature content release occurs in the mouth or in the esophagus. This particularity makes them especially suitable as chewing-capsules, or as capsules for sublingual administration, or in all cases where a fast absorption of the medicament by the mucous membrane of the mouth is desirable, as for nitroglycerine and certain steroid hormones, and particularly for those which are unstable under acidic conditions and are destroyed in the stomach.

In some cases, at low gas ratios in the wall and with thicker walls, the disintegration time of foam capsules is comparable with that of conventional hard shell gelatin capsules. A longer disintegration time is also obtainable with foam capsules. In this particular case the main advantage of foam capsules is the opacity of the wall without addition of opacifying agents.

An interesting effect of the micronization of gas bubbles in the wall of foam capsules is to provide a special configuration to the outer surface of the capsule wall (juxtaposed microbubbles) which confers to the foam capsules, when using only current approved natural and synthetic dyestuffs for pharmaceuticals, special and more brilliant color shades, like opalescent or pearly, which cannot be obtained for conventional hard shell gelatin capsules with the same dyestuffs.

Foam capsules may also be useful in other fields than pharmaceutical purposes, particularly in those cases where single dosage forms with a fast disintegration would be ideal such as:
— food packaging, as for powdered instant coffee or spices;
— candy manufacturing;
— fertilization of ornamental plants and/or indoor plants;
— packing of sensitive seeds in combination with protective agents and/or fertilizers; and
— packing of single dyestuffs (or mixtures of various dyes) doses, at precise weight for quick preparation of dyestuff solutions.

The present invention is illustrated by following examples:

## Example 1

A natural transparent aqueous solution of gelatin, at a concentration of 24% weight/weight, with an initial viscosity of 300 centipoise (0.3 Pa·s) is poured into a foaming reservoir fitted with a water jacket, a centrifugal emulsifying device and an air introduction pipe.

During a first foaming step, air is introduced into the solution and micronized by the emulsifier, rotating at full speed (2,800 rpm), until an air content of 23% volume/volume, and a satisfactory micronization of the air bubbles are reached. The white opaque foam obtained has a temperature of 48°C (water jacket at 45°C), a viscosity of 800 centipoise (0.8 Pa·s) and a density of 0.8 g/cm3.

In a second step, an exit valve or faucet from the foaming reservoir is opened and the foam flows by gravity towards the dipping dish wherein it is distributed through a longitudinal slit. The foam overflow is collected and reintroduced with a peristaltic pump into the foaming reservoir where it is recirculated through the emulsifier. The foam quality regulation is assured, over several hours, by simultaneous in-line viscosity and density measurements and subsequent addition of water and/or air and/or fresh gelatin solution.

For the formation of capsule halves by the dip-molding technique, previously lubricated metallic cap and body mold pins are dipped into the gelatin foam which flows through the dipping dish and are withdrawn and lifted slowly in conventional fashion to provide even distribution of the foam film layer over the effective area of each mold pin. The coated pins are then kept stationary for a sufficient period to gellify the film layer on the pin. The capsule halves thus formed are dried by blowing with air at about 30% relative humidity and at about 30°C, and are removed from the pins, trimmed and joined together with the other halves of the capsule to provide the finished foam capsule ready for filling.

To confirm that the obtained white opaque, slightly opalescent, foam capsules obtained have the described properties, the capsules are filled with lactose and subjected to a standard disintegration test in an Erweka apparatus according to the method described in the European Phamacopeia, 2nd. Edition, 1980, Part 1, V.5.1.1.

## TABLE 1

Compared disintegration times in seconds between foam capsules and conventional gelatin capsules

| Capsule type | Wall thickness | Mean opening time (on 6 capsules) | Mean disintegration time (on 6 capsules) |
|---|---|---|---|
| Foam capsule | 0.1270 mm ($5 \times 10^{-3}$ in.) | 30 | 81 |
| Conventional capsule | 0.1016 mm ($4 \times 10^{-3}$ inch) | 82 | 195 |

As table 1 shows, in spite of a 25% thicker wall, the disintegration of the tested foam capsules is 58% faster than for conventional capsules.

### TABLE 2
### Weight evaluation

| Mean weight foam capsules | 52 mg |
|---|---|
| Normal reference capsule | 78 mg |

As shown in Table 2, manufacturing of the described foam capsules allows a gelatin saving of 33%.

### TABLE 3
### Compared drying speeds at 30°C and 30% RH
(measured is the required time for to obtain capsule walls with 20% moisture content)

| Capsule type | Tim (min.) |
|---|---|
| Foam Capsule | 24 |
| Normal ref. capsule | 38 |

Table 3 shows that the drying of the described foam capsules is 37% faster than for conventional capsules.

### Example 2
The production of the foam capsules was the same as in Example 1, but the influence of different air content values and wall thicknesses on disintegration time was checked.

### TABLE 4
Disintegration time as a function of air content and wall thickness (on every 6 capsules)

| Sample reference capsule | % of air v/v in the foam before drying | Wall thickness (mm) | Disintegration time (in sec.) |
|---|---|---|---|
| conventional standard | — | 0.1016 | 195 |
| Foam 1 | 15% | 0.1016 | 149 |
| Foam 2 | 15% | 0.1397 | 173 |
| Foam 3 | 20% | 0.1397 | 140 |
| Foam 4 | 23% | 0.1270 | 81 |
| Foam 5 | 23% | 0.1524 | 108 |
| Foam 6 | 26% | 0.1651 | 96 |
| Foam 7 | 29% | 0.1397 | 64 |

The above demonstrates that with a suitable choice of the air proportion in the capsule wall and of the wall thickness, it is possible, within certain limits to control the disintegration time.

### Example 3
The production of colored foam capsules was the same as in Example 1, but, before foam generation, the following dyes or pigments were mixed with the gelatin solution at a concentration of 0.5% based upon weight of dry gelatin.

Red    :  azorubine

Blue    :  patent blue

Yellow  :  tartrazine

Black   :  black iron oxide

EP 0 110 502 B1

The colored foam capsules were opaque, and had similar disintegration times, as the corresponding white opaque capsules described in Example 1 above. In addition, the colored foam capsules were characterized by new, more brilliant, opalescentlike, color shades than for conventional capsules.

## Example 4
The production of flavored capsules was the same as in example 1 but, before foam generation, peppermint oil as a flavoring agent was added at a concentration of 0.6% based upon the weight of dry gelatin.

## Example 5
Example 1 was repeated by adding 2% of glycerol, based on dry gelatin weight, in the gelatin solution. The additive did not affect the ability to generate a suitable foam, and the disintegration time of the obtained capsules remained similar.

## Example 6
Example 1 was repeated by adding 0.2% sodium lauryl sulphate and 0.2% sorbitan monooleate, based on dry gelatin weight, in the gelatin solution. The additives did not affect the disintegration time of the obtained capsules, but increased notably the lifetime of the gelatin foam circulating in the dish.

## Example 7
Example 1 was repeated by replacing the air with nitrogen. This replacement did not affect the ability to generate a suitable foam, and the disintegration time of the obtained capsules remained similar.

## Claims

1. A process for the production by the dip moulding technique of a cap portion and/or body portion suitable for forming a foam capsule with telescopically engageable body and cap portions, which process comprises the following steps:—

(A) dissolving gelatin in water to make an aqueous gelatin solution, such that the weight of gelatin in the aqueous gelatin solution is in the range from 10 to 50% by weight of the solution, and the pH of the gelatin solution is in the range from 3.0 to 10.0;

(B) introducing a gas into the aqueous gelatin solution to make a gas-containing aqueous gelatin solution;

(C) micronizing the gas in the gas-containing gelatin solution to make a micronized gas-containing aqueous gelatin solution; and

(D) forming by the dip moulding technique foam capsule body and/or cap portion(s) from the micronized gas-containing aqueous gelatin solution.

2. A process according to claim 1, wherein in step B the gas is carbon dioxide, oxygen, air, nitrogen, argon or a mixture thereof; and is preferably air.

3. A process according to claim 1 or 2, wherein in step C the micronizing of the gas and aqueous gelatin solution is accomplished:

mechanically by a blender, a baker's whip, a centrifugal emulsifier or a continuous pressure beater;

by simultaneous injection of a controlled quantity of the gas and the aqueous gelatin solution into a mixing head having a rotor and a stator, both of which have teeth to micronize the injected material;

by aerosol generation or ultrasonic emulsification;

by injecting the gas through micropipes, sintered glass or sintered metal; or

by passing the gas and the aqueous gelatin solution through tubes packed with polyamide fibre or steel wool;

the micronizing of the gas and the aqueous gelatin solution preferably being accomplished within a heating system to avoid premature gelling of the resulting foam, the dispersion of the micronized gas in the aqueous gelatin solution preferably being maintained at a temperature in the range from 30 to 65° Centigrade.

4. A process according to any preceding claim, wherein in step C the dispersion of micronized gas in the aqueous gelatin solution has a gas content range of from 8 to 70% by volume, preferably from 8 to 20%, a density range of from 0.3 to 1.1 grams per cubic centimetre, a viscosity range of from 200 to 2,000 centipoises (0.2 to 2 Pa·s) and gas bubbles having a diameter range from 1 and 150 μm.

5. A process according to any preceding claim, wherein in any one or more of steps A, B and C a pharmaceutically acceptable foam stabilizing agent is added in an amount of from 0.01 to 5.0% based upon the weight of the gelatin.

6. A process according to Claim 5, wherein the foam stabilizing agent is selected from:

viscosity increasing agents such as alginic acid and salts thereof, xanthan, and cellulose derivatives such as carboxymethyl cellulose, hydroxypropylcellulose, hydroxypropylmethylcellulose, microcrystalline cellulose, and mixtures thereof:

vegetable gums, such as carrageenates, pectin, agar, and mixtures thereof:

proteins such as albumin, hydrolyzed animal proteins and mixtures thereof:

non-ionic surfactants such as fatty acid alkylolamides or ethoxylates thereof, amine oxides, amine oxides with long chain substituents, optionally substituted long chain alcohols such as lauryl alcohol and sorbitol monooleate, and mixtures thereof:

esters such as sucrose esters; fatty acid esters and ethoxylates thereof, particularly polyglycerol and sorbitan derivatives thereof; glycerides and derivatives thereof such as succinyl monoglycerides; acetic-, lactic-, citric- or acetylated tartaric acid esters of monoglycerides, glycosides, ethoxylated glycosides; lanolin and its derivatives, and mixtures thereof:

anionic surfactants such as fatty alkylarylsulphonates especially sodium dodecylbenzenesulphonic acid, alcoholsulphates preferably sodium lauryl sulphate, ethersulfphates, and mixtures thereof:

metal salts such as aluminium, calcium, potassium and iron salts, and mixtures thereof; and pharmaceutically acceptable combinations of the foregoing compounds.

7. A process according to any preceding claim, wherein in any one or more of steps A, B and C a pharmaceutically acceptable colouring agent is added in an amount of from 0.001 to 10.0% based upon the weight of the gelatin, the colouring agent preferably being an azo-dye, an iron oxide, or a natural dye.

8. A process according to any preceding claim, wherein in any one or more of steps A, B and C a pharmaceutically acceptable flavouring agent is added.

9. A process according to any preceding claim, wherein in any one or more of steps A, B and C a pharmaceutically acceptable plasticizer is added in an amount of from 0.2 to 15.0% based upon the weight of the gelatin, the plasticizer preferably being polyethylene glycol or selected from low molecular weight organic plasticizers comprising glycerol, sorbitol, dioctyl-sodium sulphosuccinate, triethyl citrate, tributyl citrate, 1,2-propylene glycol, and mono-, di- and triacetates of glycerol.

10. A process according to any preceding claim, wherein in any one or more of steps A, B and C a gelatin extender is added in an amount of from 2 to 40% based upon the weight of the gelatin, the gelatin extender preferably being a protein selected from sunflower proteins, soybean, proteins, cotton seed proteins, peanut seed protein and rape seed proteins.

11. A foam capsule body portion or a foam capsule cap portion, produced by a process according to any preceding claim.

12. A telescopically joined foam capsule with cap and body portions produced by a process according to any one of Claims 1 to 11.

13. A telescopically joined foam capsule as claimed in Claim 12, with a filled material enclosed therein.

14. A foam capsule as claimed in Claim 12 or 13, or a capsule portion as claimed in Claim 11, having a wall moisture content in the range from 10 to 18%.

**Patentansprüche**

1. Verfahren zur Herstellung eines (von) Kappen- und/oder Körperteils (-teilen) für die Bildung geschäumter Kapseln mit teleskopartig in Eingriff zu bringenden Körper- und Kappenteilen nach dem Tauchformverfahren, gekennzeichnet durch folgende Stufen:

(A) Auflösen von Gelatine in Wasser zur Zubereitung einer wäßrigen Gelatinelösung, derart, daß das Gewicht der Gelatine in der wäßrigen Gelatinelösung im Bereich von 10—50 Gew.-% der Lösung liegt und sich der pH-Wert der Gelatinelösung im Bereich von 3,0—10,0 bewegt;

(B) Einleiten eines Gases in die wäßrige Gelatinelösung zur Gewinnung einer geshaltigen wäßrigen Gelatinelösung;

(C) Mikronisieren des Gases in der geshaltigen Gelatinelösung zur Gewinnung einer mikronisiertes Gas enthaltenden wäßrigen Gelatinelösung und

(D) Ausbilden eines (von) geschäumten Kapselkörper und/oder -kappenteils (-teilen) aus der das mikronisierte Gas enthaltenden wäßrigen Gelatinelösung nach dem Tauchformverfahren.

2. Verfahren nach Anspruch 1, dadurch gekennzeichnet, daß in Stufe (B) das Gas aus Kohlendioxid, Sauerstoff, Luft, Stickstoff, Argon oder einem Gemisch derselben, vorzugsweise Luft, besteht.

3. Verfahren nach Anspruch 1 oder 2, dadurch gekennzeichnet, daß in Stufe (C) die Mikronisierung des Gases und der wäßrigen Gelatinelösung

auf mechanischem Wege mittels eines Mischers, eines Schlagbesens, einer Zentrifugenemulgiervorrichtung oder einer kontinuierlich arbeitenden Druckschlagvorrichtung,

durch gleichzeitiges Einführen einer gesteuerten Gasmenge und der wäßrigen Gelatinelösung in einen Mischkopf mit Rotor und Stator, welche beide zur Mikronisierung des zugeführten Materials mit Zähnen ausgestattet sind,

durch Aerosolerzeugung oder Ultraschallemulgieren, durch Einblasen des Gases durch Mikroröhren, gesintertes Glas der gesintertes Metall oder

durch Hindurchleiten des Gases und der wäßrigen Gelatinelösung durch mit Polyamidfasern oder Stahlwolle gepackte Röhren

bewerkstelligt wird,

wobei die Mikronisierung des Gases und der wäßrigen Gelatinelösung vorzugsweise in einem Heizsystem zur Vermeidung einer vorzeitigen Gelierung des gebildeten Schaums erfolgt und wobei die Dispersion des mikronisierten Gases in der wäßrigen Gelatinelösung vorzugsweise auf einer Temperatur im Bereich von 30°C bis 65°C gehalten wird.

4. Verfahren nach einem der vorhergehenden Ansprüche, dadurch gekennzeichnet, daß in Stufe (C) die Dispersion des mikronisierten Gases in der wäßrigen Gelatinelösung einen Gasgehaltbereich von 8—70 Vol.-%, vorzugsweise 8—20 Vol.-%, einen Dichtebereich von 0,3—1,1 g/cm$^3$, einen Viskositätsbereich von 200—2000 Centipoise (0,2—2 Pa·s) und Gasblasen eines Durchmesserbereichs von 1—150 µm aufweist.

5. Verfahren nach einem der vorhergehenden Ansprüche, dadurch gekennzeichnet, daß in einer oder mehreren der Stufen (A), (B) und (C) ein pharmazeutisch akzeptables Schaumstabilisierungsmittel in einer Menge von 0,01 bis 5,0%, bezogen auf das Gewicht der Gelatine, zugesetzt wird.

6. Verfahren nach Anspruch 5, dadurch gekennzeichnet, daß das Schaumstabiliserungsmittel aus:

viskositätserhöhenden Mitteln, wie Alginsäure und deren Salzen, Xanthan und Cellulosederivaten, z.B. Carboxymethylcellulose, Hydroxypropylcellulose, Hydroxypropylmethylcellulose, mikrokristalliner Cellulose und Mischungen derselben,

Pflanzengummis, z.B. Karrageenaten, Pektin, Agar und Mischungen derselben,

Proteinen, z.B. Albumin, hydrolysierten Tierproteinen und Mischungen derselben,

nicht-ionischen Netzmitteln, z.B. Fettsäurealkylolamiden oder deren Ethoxylaten, Aminoxiden, Aminoxiden mit langkettigen Substituenten, gegebenenfalls substituierten langkettigen Alkoholen, z.B. Laurylalkohol und Sorbitmonooleat, sowie Mischungen derselben, Estern, z.B. Saccharoseestern, Fettsäureestern und deren Ethoxylaten, insbesondere deren Polyglycerin- und Sorbitanderivaten, Glyceriden und Derivaten der selben, z.B. Succinylmonoglyceriden, Essig-, Milch-, Zitronen- oder acetylierten Weinsäureestern von Monoglyceriden, Glycosiden, ethoxylierten Glycosiden, Lanolin und deren Derivaten, sowie Mischungen derselben, anionischen Netzmitteln, z.B. Fettsäurealkylarylsulfonaten, insbesondere Natriumdodecylbenzolsulfonsäure, Alkoholsulfaten, vorzugsweise Natriumlaurylsulfat, Ethersulfaten und Mischungen derselben, Metallsalzen, z.B. Aluminium-, Calcium-, Kalium- und Eisensalzen und deren Mischungen und pharmazeutisch akzeptablen Kombinationen der genannten Verbindungen, ausgewählt ist.

7. Verfahren nach einem der vorhergehenden Ansprüche, dadurch gekennzeichnet, daß in einer oder mehreren der Stufen (A), (B) und (C) ein pharmazeutisch akzeptables Färbemittel in einer Menge von 0,001—10,0%, bezogen auf das Gewicht der Gelatine, zugesetzt wird, wobei das Färbemittel vorzugsweise aus einem Azofarbstoff, Eisenoxid oder seinem natürlichen Farbstoff besteht.

8. Verfahren nach einem der vorhergehenden Ansprüche, dadurch gekennzeichnet, daß in einer oder mehreren der Stufen (A), (B) und (C) ein pharmazeutisch akzeptabler Geschmacksstoff zugesetzt wird.

9. Verfahren nach einem der vorhergehenden Ansprüche, dadurch gekennzeichnet, daß in einer oder mehreren der Stufen (A), (B) und (C) ein pharmazeutisch akzeptables Plastifizierungsmittel in einer Menge von 0,2—15,0%, bezogen auf das Gewicht der Gelatine, zugesetzt wird, wobei das Plastifizierungsmittel vorzugsweise aus Polyethylenglykol besteht oder aus einem niedrigmolekularen organischen Plastifizierungsmittel in Form von Glycerin, Sorbit, Dioctyl-natriumsulfosuccinat, Triethylcitrat, Tributylcitrat, 1,2-Propylenglykol und Mono-, Di- und Triacetaten von Glycerin ausgewählt ist.

10. Verfahren nach einem der vorhergehenden Ansprüche, dadurch gekennzeichnet, daß in einer oder mehreren der Stufen (A), (B) und (C) ein Gelatinestreckmittel in einer Menge von 2—40%, bezogen auf das Gewicht der Gelatine, zugesetzt wird, wobei das Gelatinestreckmittel vorzugsweise aus einem Protein, ausgewählt aus Sonnenblumenproteinen, Sojabohnenproteinen, Baumwollsaatproteinen, Erdnußsaatproteinen und Rapssaatproteinen, besteht.

11. Geschäumter Kapselkörperteil oder geschäumter Kapselkappenteil, hergestellt nach dem Verfahren nach einem der vorhergehenden Ansprüche.

12. Teleskopartig vereinigte geschäumte Kapsel mit Kappen- und Körperteilen, die nach einem Verfahren gemäß einem der Ansprüche 1 bis 11 hergestellt wurden.

13. Teleskopartig vereinigte geschäumte Kapsel nach Anspruch 12 mit einer darin befindlichen Füllung.

14. Geschäumte Kapsel nach Ansprüchen 12 oder 13 oder ein Kapselteil nach Anspruch 11 mit einem Wandfeuchtigkeitsgehalt im Bereich von 10—18%.

## Revendications

1. Un procédé de production par la technique de moulage par immersion d'une partie formant couvercle et/ou d'une partie formant corps convenant à la formation de capsules alvéolaires formées d'une partie formant couvercle et d'une partie formant corps téléscopiquement engageables l'une dans l'autre, ce procédé comprenant les étapes suivantes:

(A) dissolution de la gélatine dans de l'eau pour former une solution aqueuse de gélatine, de sorte que le poids de gélatine dans la solution aqueuse de gélatine soit compris entre 10 et 50% en poids de la solution et que le pH de la solution de gélatine soit compris entre 3,0 et 10,0;

(B) introduction d'un gaz dans la solution aqueuse de gélatine pour former une solution de gélatine aqueuse contenant un gaz;

(C) micronisation du gaz dans la solution de gélatine contenant le gaz pour former une solution de gélatine aqueuse contenant un gaz micronisé et;

(D) formation par la technique du moulage par immersion de portion(s) formant couvercle(s) et/ou corps d'une capsule alvéolaire à partir de la solution aqueuse de gélatine contenant un gaz micronisé.

9

# EP 0 110 502 B1

2. Un procédé selon la revendication 1, dans lequel dans l'étape B, le gaz consiste en dioxyde de carbone, oxygène, air, azote, argon ou un de leurs mélanges et de préférence de l'air.

3. Un procédé selon la revendication 1 ou la revendication 2, dans lequel dans l'étape C, la micronisation du gaz et de la solution de gélatine aqueuse est obtenue:

par voie mécanique à l'aide d'un mélangeur, d'un fouet mécanique, d'un émulsificateur centrifuge ou d'un batteur sous pression continue;

par injection simultanée d'une quantité contrôlée de gaz et de la solution aqueuse de gélatine dans une tête de mélange comprenant un rotor et un stator qui tous deux comportent des dents pour microniser la matière injectée;

par production d'aérosol ou émulsification ultrasonique;

par injection du gaz par des micro-conduits de verre fritté ou de métal fritté ou par passage du gaz et de la solution aqueuse de gélatine dans des tubes remplis d'un garnissage de fibres polyamides ou de laine de verre;

la micronisation du gaz et de la solution aqueuse de gélatine étant de préférence effectuées dans un système chauffé pour éviter un gel prématuré de la mousse résultante, la dispersion du gaz micronisé dans la solution aqueuse de gélatine étant de préférence maintenue à une température comprise entre 30 et 65°C.

4. Un procédé selon une quelconque des revendications précédentes dans lequel dans l'étape C, la dispersion du gaz micronisé dans la solution aqueuse de gélatine présente une teneur en gaz comprise entre 8 et 70% en volume, de préférence 8 et 20%, une fourchette de densité comprise entre 0,3 et 1,1 g/$cm^3$, un domaine de viscosité compris entre 200 et 2000 centipoises (0,2 à 2 Pa·s) et des bulles de gaz présentant un diamètre compris entre 1 et 150 μm.

5. Un procédé selon une quelconque des revendications précédentes dans lequel, dans l'une ou plusieurs des étapes A, B et C un agent stabilisant de mousse pharmaceutiquement acceptable est ajouté en une quantité comprise entre 0,01 et 5,0% exprimés par rapport au poids de la gélatine.

6. Un procédé selon la revendication 5 dans lequel, l'agent stabilisateur de la mousse est choisi parmi:

les agents augmentant la viscosité telle que l'acide alginique et ses sels, le xanthane et les dérivés de cellulose tels que, carboxyméthylcellulose, hydroxypropylcellulose, hydroxypropylméthylcellulose, microcrystalline et ses mélanges;

des gommes végétales telles que carragénate, pectine, agar et leurs mélanges;

des protéines telles que albumine, les protéines animales hydrolisées et leurs mélanges;

les agents tensio-actifs nonioniques tels que les alkylolamides d'acide gras ou leurs éthoxylates, les oxydes d'amine, des oxydes amine présentant des substituants à longue chaîne éventuellement substitués par des alcools à longue chaîne tels que l'alcool laurylique et le monooléate de sorbitol ainsi que leurs mélanges;

les esters tels que les esters de sucrose, des esters d'acides gras et leurs éthoxylates, notamment, le polyglycérol et les dérivés de sorbitant en dérivant, des glycérides et leurs dérivés tels que succinyle monoglycérides; des esters de monoglycéride de glycoside ou de glycoside éthoxylé d'acide acétique, lactique citrique ou acide tartrique acétylé; la lanoline et ses dérivés et leurs mélanges;

des agents tensio-actifs tels que alkylarylsulfonate gras notamment dodécylbenzènesulfonate de sodium, des alcoolsulfates, de préférence le laurylsulfate de sodium, les étersulfates et leurs mélanges;

des sels de métaux tels que des sels d'aluminium, de calcium, de potassium et de fer et leurs mélanges et les combinaisons pharmaceutiquement acceptables des composés précités.

7. Un procédé selon une quelconque des revendications précédentes dans lequel, dans une ou plusieurs des étapes A, B et C un agent de coloration pharmaceutiquement acceptable est ajouté en une proportion comprise entre 0,01 et 10% en poids exprimés par rapport au poids de la gélatine, cet agent de coloration étant de préférence un colorant azo, un oxyde de fer ou un colorant naturel.

8. Un procédé selon une quelconque des revendications précédentes dans lequel, dans une ou plusieurs des étapes A, B et C est ajouté un agent conférant du groupe du goût pharmaceutiquement acceptable.

9. Un procédé selon une quelconque des revendications précédentes dans lequel, dans une ou plusieurs des étapes A, B et C est ajouté un plastifiant pharmaceutiquement acceptable en une quantité comprise entre 0,2 et 15% en poids exprimés par rapport au poids de la gélatine, ce plastifiant consistant de préférence en polyéthylène glycol ou étant choisi parmi les plastifiants organiques de bas poids moléculaire consistant en glycérol, sorbitol, sulfosuccinate de dioctylsodium, citrate de triéthyle, citrate de tributyle, 1,2-propylèneglycol et mono di et triacétate de glycérol.

10. Un procédé selon une quelconque des revendications précédentes dans lequel, dans une ou plusieurs des étapes A, B et C est ajouté un agent d'extension de gélatine en une quantité comprise entre 2 et 40% exprimés par rapport au poids de gélatine, cet agent d'extension de gélatine consistant de préférence en une protéine choisie parmi les protéines du tournesol, les protéines du soja, les protéines des graines de coton, les protéines des graines de cacahuète et les protéines de graine de colza.

11. Une portion de corps de capsule alvéolaire produite par un procédé selon une quelconque des revendications précédentes.

12. Une capsule alvéolaire formée de portions, de couvercle et de corps, jointes télescopiquement, produites par un procédé selon l'une quelconque des revendications 1 à 11.

10

13. Une capsule alvéolaire jointe télescopiquement, ainsi que revendiqué dans la revendication 12, contenant une matière incluse.

14. Une capsule alvéolaire, ainsi que revendiqué dans la revendication 12 ou 13 ou une partie de capsule, telle que revendiquée dans la revendication 11, présentant une teneur en humidité des parois comprise entre 10 et 18%.